# EUROPÄISCHE PATENTANMELDUNG

(11) **EP 2 457 565 A1**
(43) Veröffentlichungstag der Anmeldung: **30.05.2012**
(21) Anmeldenummer: 10015079.6
(22) Anmeldetag: 29.11.2010
(51) Int. Cl.: A61K 9/70, A61K 31/381

(54) **Transdermales therapeutisches System enthaltend Rotigotin**

(71) Anmelder: Ratiopharm GmbH, 89079 Ulm (DE)
(72) Erfinder: Drescher, Christian, 89073 Ulm (DE)

(57) **Zusammenfassung**

Die vorliegende Erfindung betrifft ein den Wirkstoff Rotigotin enthaltendes Pflaster zur transdermalen Abgabe des pharmazeutischen Wirkstoffs Rotigotin, umfassend eine Trägerschicht (1), eine den Wirkstoff enthaltenden Matrixschicht (2), eine vor dem Aufbringen auf die Haut zu entfernenden, abziehbaren Schutzfolie (4), dadurch gekennzeichnet, dass zwischen der Matrixschicht (2) und der abziehbaren Schutzfolie (4) eine zusätzliche Zwischenschicht (3) flächendeckend angebracht ist.

## Beschreibung

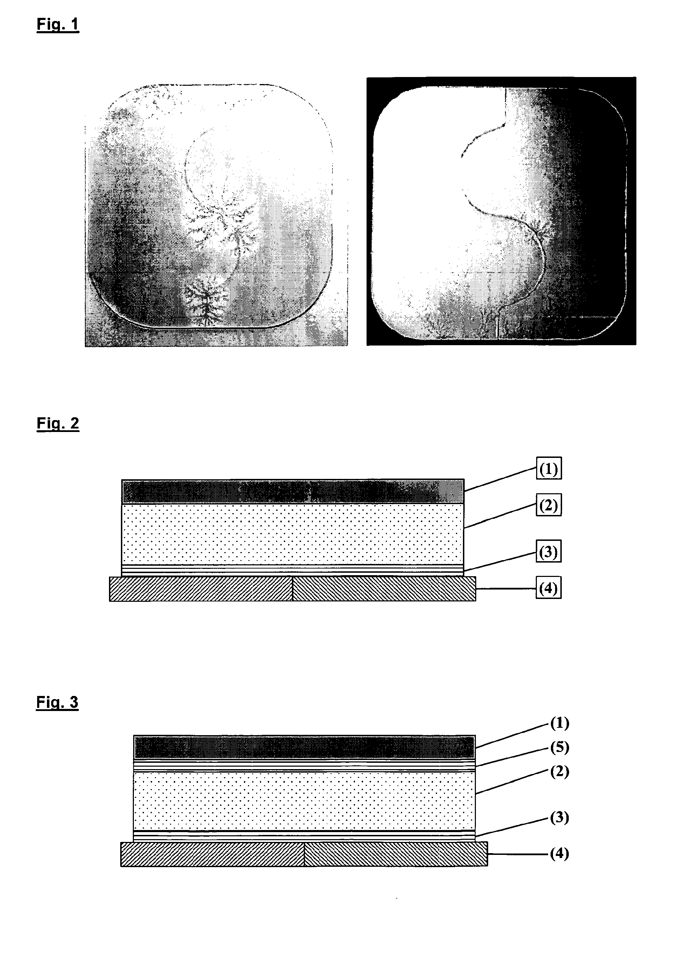

Rotigotin [(-)-5, 6,7, 8-Tetrahydro-6-[propyl [2-(2-thienyl) ethyl] amino]-1-naphtol] ist ein pharmazeutischer Wirkstoff, der in der Behandlung von u.a. Morbus Parkinson eingesetzt wird. Das einzige zur Zeit am Markt befindliche rotigotinhaltige Arzneimittel ist ein transdermales therapeutisches System (TTS). Neupro^{®} von der Firma UCB enthält den Wirkstoff eingebettet in eine Polymermatrix, und zwar in einer Menge oberhalb der Sättigungskonzentration. Damit stellt es ein sog. "übersättigtes" System dar. Übersättigte Wirkstoff/Polymer- Systeme sind bekanntermaßen metastabil, d.h. unter ungünstigen Bedingungen kann Wirkstoff auskristallisieren: So mußte durch eine Rückrufaktion für NEUPRO^{®} gestartet werden, weil aus den Plastern Wirkstoff auskristallisierte (vgl. Fig. 1). Auskristallisierter Wirkstoff kann eine Vielzahl von Problemen verursachen: Zum Beispiel ist weniger Wirkstoff zur Absorption durch die Haut verfügbar, wodurch die beabsichtigte Verabreichungsrate nicht mehr gewährleistet werden kann. Auch kann auskristallisierter Wirkstoff die Klebeeigenschaften (Adhäsion bzw. Kohäsion) der Klebematrix negativ beeinflussen. Noch heute ist für Transport und Lagerung für NEUPRO® eine Kühlkette erforderlich und es wird empfohlen, NEUPRO^{®} im Kühlschrank aufzubewahren.

Im Stand der Technik, z.B. WO 99/49852 oder WO 2004/058247 werden für die Herstellung von Rotigotin-Pflastern sog. Matrix-Systeme vorgeschlagen, bei denen der Wirkstoff in eine Matrix aus druckempfindlichem Polymerklebstoff ("Pressure sensitive adhesive") eingearbeitet wird. Bei der Auswahl der zu verwendenden Polymer-Haftkleber muss dabei stets ein Kompromiss eingegangen werden, da der Kleber eine ausreichende Löslichkeit für den Wirkstoff und gleichzeitig zufrieden stellende Klebeeigenschaften aufweisen muss. Noch verkompliziert wird die Entwicklung eines geeigneten TTS dadurch, dass Wirkstoffe in höheren Konzentrationen (Neupro® beispielsweise enthält Rotigotin-Base in einem Anteil von rund 9 Gew. %) die Klebeeigenschaften von Polymer-Matrizes erheblich beeinflussen können - so kann es zu Problemen mit der Adhäsion, der Kohäsion sowie zum Auftreten des Phänomens des "kalten Flusses" kommen. Selbstklebende Polymere enthalten darüber hinaus oft freie funktionelle Gruppen wie Hydroxyl- (OH-) oder Carboxyl- (COOH-) Gruppen, welche mit dem Wirkstoff wechselwirken und dadurch dessen Kristallisations- sowie Freisetzungsverhalten ungünstig beeinflussen können.

Ausgehend vom Stand der Technik stellt sich die Aufgabe, ein Rotigotin-TTS bereitzustellen, welches die unabhängige Optimierung der Klebeeigenschaften sowie des Freisetzungsverhaltens ermöglicht.

Diese Aufgabe wurde gelöst durch die Bereitstellung eines den Wirkstoff Rotigotin enthaltendes Transdermales therapeutisches System (TTS) mit einer wirkstoffundurchlässige Trägerschicht (1), einer den Wirkstoff enthaltenden Matrixschicht (2), einer vor dem Aufbringen auf die Haut zu entfernenden, abziehbaren Schutzfolie (4), dadurch gekennzeichnet, dass zwischen der Matrixschicht (2) und der abziehbaren Schutzfolie (4) eine Zwischenschicht (3) flächendeckend angebracht ist.

Während die Matrixschicht (2) die Freisetzungseigenschaften für den Wirkstoff bestimmt, werden die Klebeeigenschaften des TTS durch geeignete Wahl der Zwischenschicht (3) determiniert. Durch den erfindungsgemäßen Aufbau des TTS können so die Freisetzungseigenschaften für den Wirkstoff sowie die Klebeeigenschaften des Pflasters unabhängig voneinander moduliert werden.

Da bei einem solchen System für die Matrixschicht (2) auch nicht-selbstklebende Polymere verwendet werden können, sind zudem die Auswahlmöglichkeiten der für den Vorrat verwendbaren Polymere deutlich erhöht. Nicht-selbstklebende Polymere im Sinne dieser Erfindung sind solche, deren Klebkraft nicht ausreicht, so dass ein erfindungsgemäßes TTS über den gesamten Verabreichungszeitraum an der menschlichen Haut - ohne weitere Hilfsmittel - haften bleibt. Beispiele für nicht-selbstklebende Polymere sind Polymere mit längeren aliphatischen Ketten und/oder solche ohne funktionelle Gruppen.

Funktionelle Gruppen wie beispielsweise Carboxylgruppen, welche in der Regel für die klebenden Eigenschaften eines Polymers verantwortlich sind, können mit basischen Wirkstoffen wie dem Rotigotin auf ungewünschte Art und Weise interagieren, z.B. durch Salzbildung. Hingegen können Polymere ohne diese funktionelle Gruppen bevorzugte Lösungs- sowie Freisetzungseigenschaften für den Wirkstoff aufweisen.

Eine bevorzugte Ausführungsform der Erfindung betrifft daher ein TTS mit einer wirkstoffundurchlässigen Trägerschicht (1), einer den Wirkstoff enthaltenden Matrixschicht (2), einer vor dem Aufbringen auf die Haut zu entfernenden, abziehbaren Schutzfolie (4) und einer zwischen der Matrixschicht (2) und der abziehbaren Schutzfolie (4) flächendeckend angebrachten Zwischenschicht (3), dadurch gekennzeichnet, dass die Matrixschicht (2) aus einem nicht selbstklebenden Polymer besteht.

Ein weiterer Vorteil der Erfindung ist es, dass für die Zwischenschicht (3) auch solche Polymere verwendet werden, die aufgrund einer sehr geringen Löslichkeit für Rotigotin als Material für den Vorrat (2) nicht ohne Weiteres in Betracht kommen.

Fig. 2 zeigt den schematischen Aufbau eines TTS einer selbstklebenden Matrixschicht, enthaltend
- Eine wirkstoffundurchlässige Trägerschicht (1)
- Eine Rotigotin enthaltende, selbstklebende Matrixschicht (2)
- Eine zwischen der Matrixschicht (2) und der abziehbaren Schutzfolie (4) flächendeckend angebrachte Zwischenschicht (3)
- Mindestens eine vor dem Aufbringen auf die Haut zu entfernenden, abziehbaren Schutzfolie (4)

Fig. 3 zeigt den schematischen Aufbau eines TTS mit nicht-klebender Matrixschicht, enthaltend
- Eine wirkstoffundurchlässige Trägerschicht (1)
- Eine Rotigotin enthaltende Matrixschicht, nicht klebend (2)
- Eine erste, zwischen der Matrixschicht (2) und der abziehbaren Schutzfolie (4) flächendeckend angebrachte Zwischenschicht (3)
- Mindestens eine vor dem Aufbringen auf die Haut zu entfernenden, abziehbaren Schutzfolie (4)
- Eine zweite, zwischen der Matrixschicht (2) und der Trägerschicht (1) flächendeckend angebrachte Zwischenschicht (5)

Die flächendeckende Abschirmung der Matrixschicht (2) durch die Zwischenschicht (3) hat zudem den zusätzlichen Vorteil, dass ein direkter Kontakt des Reservoirs mit den Schnittkanten vor dem Aufbringen auf die Haut zu entfernenden, abziehbaren Schutzfolie (4) vermieden wird, was beim Marktprodukt Neupro® offensichtlich zur Kristallbildung führte (siehe Fig. 1).

Die wirkstoffundurchlässige Trägerschicht (1) des erfindungsgemäßen Pflasters ist für den Wirkstoff sowie die in der wirkstoffhaltigen Schicht enthaltenen Verbindungen undurchlässig und inert, d.h. es findet auch keine Migration in die Trägerschicht statt. Die wirkstoffundurchlässige Trägerschicht kann Luft- und wasserdurchlässig sein, ist allerdings vorzugsweise okklusiv. Sie muss einerseits eine gewisse Steifigkeit aufweisen, da sie die wirkstoffhaltige Matrixschicht (2) ausreichend stabilisiert, so dass das TTS handhabbar ist, muss andererseits aber auch eine gewisse ausreichende Flexibilität aufweisen, damit sich auch größere TTS Pflaster noch der Haut anpassen können, damit ein ausreichender Tragekomfort gewährleistet ist.

Die wirkstoffundurchlässige Trägerschicht (1) besteht aus Polymeren wie Polyestern (beispielsweise PET), Polyolefinen (beispielsweise PE), Polycarbonaten, Polyethylenoxiden, Polyurethanen, Polystyrolen, Polyamiden, Polyvinylacetaten sowie Polyvinylchloriden. Bevorzugt ist eine mit Aluminium bedampfte PE/PET-Trägerschicht (beispielsweise Scotchpak 1109, 3M).

Als Materialien für die Rotigotin enthaltende Matrixschicht (2) werden filmbildende Polymere verwendet wie z.B. Polyacrylate, Ethylen/Ethylacrylat Copolymere, Poliisobutylene (PIB), sowie Polysiloxane. Bevorzugt sind AcrylatCopolymere, beispielsweise solche vom Typ Eudragit®.

Das Rotigotin wird bevorzugt in Form der freien Rotigotin-Base eingestzt.

Die wirkstoffhaltige Matrixschicht (2) kann außer dem pharmazeutischen Wirkstoff Rotigotin noch Hilfsstoffe wie Antioxidantien, Weichmacher, Permeationsförderer, Lösungsvermittler, Vernetzer, Emulgatoren, Konservierungsmittel und/oder Verdickungsmittel enthalten.

Der Wirkstoff kann in der Matrixschicht (2) gelöst und/oder dispergiert vorliegen. Vorzugsweise liegt der Wirkstoff komplett in gelöster Form vor.

Die Löslichkeit der Matrixschicht (2) für den Wirkstoff Rotigotin liegt zwischen 4 und 30 Gew.-% auf, vorzugsweise zwischen 6 und 20 Gew.-%, noch weiter bevorzugt zwischen 8 und 15 Gew.-%.

Der Wirkstoffgehalt an Rotigotin in der Matrixschicht (2) liegt bei 4 bis 30 Gew.-%, bevorzugt 6 bis 20 Gew.-%, noch weiter bevorzugt bei 8 bis 15 Gew.-%, bezogen auf das Gesamtgewicht der Matrixschicht.

Für die Zwischenschicht (3) eignen sich Polymere auf der Basis von Silikonen, Polyacrylaten, Polyvinylethern, Polyisobutylenen (PIB), Styrol-Isopren-oder Butadien-Styrol-Copolymeren, Polyolefinen, Polyvinylacetaten, Polyamiden und/oder EthylenVinylacetat-Copolymeren.

Die Zwischenschicht (3) ist vorzugsweise selbstklebend und besteht bevorzugt aus einem druckempfindlichen Polymerklebstoff ("Pressure sensitive adhesive"), bevorzugt aus einem aminresistenten Silikonkleber wie z.B. BIO-PSA Q7-4301 oder BIO-PSA Q7-4201 (Dow Corning).

Die Zwischenschicht (3) kann optional zusätzlich Hilfsstoffe wie Weichmacher, Füllstoffe, Vernetzer, Konservierungsmittel und/oder Lösungsmittel sowie Antioxidantien enthalten.

Die Dicke der Zwischenschicht (3) beträgt zwischen 4 und 25µm, bevorzugt zwischen 8 und 20µm und besonders bevorzugt zwischen 10 und 15µm.

Die Zwischenschicht kann optional auch gleichzeitig als Kontrollmembran für den Wirkstofffluss dienen. Dabei wird die Permeation des Wirkstoffes um nicht mehr als 20%, vorzugsweise nicht mehr als 10%, reduziert.

Die Zwischenschicht soll außer ihrer primären Funktion, nämlich die Regulierung der Klebkraft, möglichst wenig mit dem Wirkstoff interagieren. Die Löslichkeit der Zwischenschicht für den Wirkstoff beträgt deshalb vorzugsweise nur 5% bis 15% der Löslichkeit der Matrixschicht, bevorzugt 5% bis 10%. Das Verhältnis der Dicke der Matrixschicht zu der Dicke der Zwischenschicht beträgt zwischen 20:1 und 3:1, bevorzugt zwischen 10:1 und 5:1.

Die vor dem Aufbringen auf die Haut zu entfernende, abziehbare Schutzfolie (4) besteht aus Polyethylen, Polypropylen, Polyestern (PET), Polysiloxanen, Polyvinylchlorid oder Polyurethan oder gegebenenfalls aus behandelten Papierfasern und ist bevorzugterweise silikon-, fluorsilikon- oder fluorcarbon- polymerbeschichtet. Bevorzugt ist fluorpolymerbeschichteter PET-Liner (Beispielsweise Scotchpak 1022, 3M).

Für die zweite Zwischenschicht (5) eignen sich prinzipiell die gleichen Materialien wie für die erste Zwischenschicht (3). Vorzugsweise bestehen beide aus dem selben Material.

Das TTS weist vorzugsweise einen Aufbau auf wie in Fig. 1 oder Fig. 2 dargestellt. Das TTS kann aber zusätzlich eine klebende Folie ("Overtape") aufweisen, mit welchem das TTS auf der Haut fixiert wird.

Das TTS hat eine Größe von 5 bis 100 cm², bevorzugt 10 bis 50 cm².

Das Flächengewicht des TTS (ohne die abziehbare Schutzfolie) beträgt 20 bis 150 g/cm², bevorzugt 30 bis 100 g/cm², besonders bevorzugt 40 bis 70 g/cm².

In einer bevorzugten Ausführungsform hat das TTS eine Klebkraft von zwischen 5 N/25 mm² und 100 N/25 mm², bevorzugt zwischen 10 N/25 mm² und 50 N/25 mm². Die Bestimmung der Klebkraft erfolgt mittels bekannter Standardverfahren (z.B. ASTM D1000-99).

Das erfindungsgemäße Pflaster eignet sich für die transdermale Verabreichung von Rotigotin über 12h bis zu 3 Tagen, das heißt der Tragezeitraum eines Pflasters erstreckt sich über diesen Zeitraum. Bevorzugt ist ein Tragezeitraum von 24h bis 48h. Bevorzugte Freisetzungsraten liegen über einen wesentlichen Teil des Verabreichungszeitraums im Bereich von 0,05 µg/h bis 1 µg/h, bevorzugt 0,1 bis 0,5 µg/h.

Das TTS ruft in einer bevorzugten Ausführungsform eine mittlere maximale Plasmakonzentration (cₘₐₓ) von Rotigotin von 0,1 bis 150 ng/mL, bevorzugt 0,5 bis 100 ng/mL, hervor. In einer weiter bevorzugten Ausführungsform erzeugt das TTS nach wiederholten Einzelapplikationen eine AUC von 4,0 bis 30 ng*h/mL, insbesondere 5,0 bis 20 ng*h/mL.

Die Herstellung des erfindungsgemäßen Pflasters erfolgt nach bekannten Herstellungsverfahren. Die Herstellung von Rotigotin-TTS im Sinne dieser Erfindung umfasst beispielsweise folgende Schritte:
a. Lösen oder Suspendieren von Rotigotin-Base in einem geeigneten Lösemittel
b. Zugeben der Lösung/Suspension zu einer Polymerlösung unter Rühren
c. Beschichten einer geeigneten Trägerfolie (1) mit der wirkstoffhaltigen Polymerlösung
d. Trocknung unter Entfernung des Lösemittels, wobei ein erstes Laminat aus Trägerfolie und rotigotin-haltiger Matrixschicht (2) resultiert
e. Beschichten einer geeigneten Schutzfolie (4) mit einer geeigneten Polymerlösung
f. Trocknen unter Entfernung des Lösemittels, wobei ein zweites Laminat aus Schutzfolie (4) und wirkstofffreier Zwischenschicht (3) resultiert
g. Zusammenfügen der beiden Laminate, so dass die Matrixschicht (2) und die Trennschicht (3) aufeinander zu liegen kommen (siehe Abbildungen)
h. Ausstanzen von Pflastern geeigneter Größe aus dem Gesamtlaminat
i. Verpacken der einzelnen TTS in geeignete Behältnisse

Für TTS mit nicht-selbstklebender Matrixschicht wird die Trägerfolie (1) vor dem Aufbringen der Matrixschicht (Verfahrensschritt c) zunächst mit einer geeigneten Polymerlösung beschichtet und anschließend getrocknet.

Transdermales therapeutisches System (TTS) enthaltend den Wirkstoff Rotigotin in Form der freien Base, umfassend eine wirkstoffundurchlässige Trägerschicht (1), eine den Wirkstoff enthaltende Matrixschicht (2), eine vor dem Aufbringen auf die Haut zu entfernende, abziehbare, polymerbeschichtete Schutzfolie (4) und eine zwischen der Matrixschicht (2) und der abziehbaren Schutzfolie (4) flächendeckend angebrachte Zwischenschicht (3), dadurch gekennzeichnet, dass die Matrixschicht (2) aus einem druckempfindlichen Polymerklebstoff besteht, in dem der Wirkstoff komplett gelöst vorliegt.

Transdermales therapeutisches System (TTS) enthaltend den Wirkstoff Rotigotin in Form der freien Base, umfassend eine wirkstoffundurchlässige Trägerschicht (1), eine den Wirkstoff enthaltende Matrixschicht (2), eine vor dem Aufbringen auf die Haut zu entfernende, abziehbare, polymerbeschichtete Schutzfolie (4) und eine zwischen der Matrixschicht (2) und der abziehbaren Schutzfolie (4) flächendeckend angebrachte Zwischenschicht (3), dadurch gekennzeichnet, dass die Matrixschicht (2) aus einem druckempfindlichen Polyacrylatklebstoff besteht und zwischen 8 und 15 Gew.-% komplett gelösten Wirkstoff enthält.

Transdermales therapeutisches System (TTS) enthaltend den Wirkstoff Rotigotin in Form der freien Base, umfassend eine wirkstoffundurchlässige Trägerschicht (1), eine den Wirkstoff enthaltende Matrixschicht (2), eine vor dem Aufbringen auf die Haut zu entfernende, abziehbare, polymerbeschichtete Schutzfolie (4) und eine zwischen der Matrixschicht (2) und der abziehbaren Schutzfolie (4) flächendeckend angebrachte Zwischenschicht (3), dadurch gekennzeichnet, dass das Flächengewicht des TTS zwischen 30 g/cm² und 100 g/cm² liegt und das Verhältnis der Dicke der Matrixschicht (2) zu der Dicke der Zwischenschicht (3) zwischen 10:1 und 5:1 beträgt.

Transdermales therapeutisches System (TTS) enthaltend den Wirkstoff Rotigotin in Form der freien Base, umfassend eine wirkstoffundurchlässige Trägerschicht (1), eine den Wirkstoff enthaltende Matrixschicht (2), eine vor dem Aufbringen auf die Haut zu entfernende, abziehbare, polymerbeschichtete Schutzfolie (4) und einer ersten, zwischen der Matrixschicht (2) und der abziehbaren Schutzfolie (4) flächendeckend angebrachten Zwischenschicht (3), dadurch gekennzeichnet, dass zwischen der Matrixschicht (2) und der Trägerschicht (1) eine zweite Zwischenschicht (5) flächendeckend angebracht ist.

## Patentansprüche

1. Transdermales Therapeutisches System (TTS) enthaltend den Wirkstoff Rotigotin oder eines seiner pharmazeutisch annehmbaren Salze mit einer wirkstoffundurchlässigen Trägerschicht (1), einer den Wirkstoff enthaltenden Matrixschicht (2) und einer vor dem Aufbringen auf die Haut zu entfernenden, abziehbaren Schutzfolie (4), **dadurch gekennzeichnet, dass** zwischen der Matrixschicht (2) und der abziehbaren Schutzfolie (4) eine erste Zwischenschicht (3) flächendeckend angebracht ist.

2. TTS gemäß Anspruch 1, wobei die erste Zwischenschicht (3) aus einem druckempfindlichen Polymerklebstoff besteht.

3. TTS gemäß Anspruch 1, wobei die Matrixschicht (2) aus nicht-klebenden Polymeren mit längeren aliphatischen Ketten, bevorzugt Polyacrylat-Estern mit 4 bis 8 C-Atomen besteht und eine zweite Zwischenschicht (5) aufweist, die aus dem selben Material wie die erste Zwischenschicht (3) besteht.

4. TTS gemäß einem der vorhergehenden Ansprüche, wobei die Dicke der Zwischenschicht bzw. Zwischenschichten jeweils zwischen 4 µm und 25 µm, bevorzugt zwischen 8 µm und 20 µm und besonders bevorzugt zwischen 10 µm und 15 µm liegt.

5. TTS gemäß einem der vorhergehenden Ansprüche, wobei es sich bei dem Wirkstoff um die freie Rotigotin-Base handelt.

6. TTS gemäß einem der vorhergehenden Ansprüche, wobei der Wirkstoff komplett in der Matrixschicht (2) gelöst vorliegt.

7. TTS gemäß einem der vorhergehenden Ansprüche, wobei die Löslichkeit der Zwischenschicht für den Wirkstoff zwischen 4 und 30 Gew.-%, vorzugsweise zwischen 6 und 20 Gew.-%, noch weiter bevorzugt zwischen 8 und 15 Gew.-% beträgt.

8. TTS gemäß einem der vorhergehenden Ansprüche, wobei der Wirkstoffgehalt an Rotigotin, bezogen auf die freie Base, in der Matrixschicht (2) zwischen 4 bis 30 Gew.-%, bevorzugt 6 bis 20 Gew.-%, noch weiter bevorzugt 8 bis 15 Gew.-% liegt.

9. TTS gemäß einem der vorhergehenden Ansprüche, wobei die Löslichkeit der Zwischenschicht bzw. Zwischenschichten für den Wirkstoff 5% bis 15%, bevorzugt 5% bis 10%, der Löslichkeit der Matrixschicht für den Wirkstoff beträgt.

10. TTS gemäß einem der vorhergehenden Ansprüche, wobei das Verhältnis der Dicke der Matrixschicht (2) zu der Dicke der ersten Zwischenschicht (3) zwischen 20:1 und 3:1, bevorzugt zwischen 10:1 und 5:1 liegt.

11. TTS gemäß einem der vorhergehenden Ansprüche, wobei das TTS eine Größe von 5 cm² bis 100 cm², bevorzugt 10 cm² bis 50 cm² aufweist.

12. TTS gemäß einem der vorhergehenden Ansprüche, wobei das TTS eine Klebkraft von zwischen 5 N/25 mm² und 100 N/25 mm², bevorzugt zwischen 10 N/25 mm² und 50 N/25 mm² aufweist.

13. TTS gemäß einem der vorhergehenden Ansprüche, wobei das TTS für eine Verabreichung von Rotigotin über 12 h bis 3 Tage, bevorzugt über 24 h bis 48 h geeignet ist.

14. TTS gemäß einem der vorhergehenden Ansprüche, wobei die Freisetzungsrate einen wesentlichen Teil des Verabreichungszeitraums im Bereich von 0,05 µg/h bis 1 µg/h, bevorzugt 0,1 µg/h bis 0,5 µg/h liegt.

15. TTS gemäß einem der vorhergehenden Ansprüche, wobei das TTS über einen wesentlichen Teil des Verabreichungszeitraums eine mittlere maximale Plasmakonzentration (cₘₐₓ) von Rotigotin von 0,1 ng/mL bis 150 ng/mL, bevorzugt 0,5 ng/mL bis 100 ng/mL erzeugt.
